Europäisches Patentamt

**European Patent Office**

Office européen des brevets

(11) Veröffentlichungsnummer: **0 282 473 B1**

(12) **EUROPÄISCHE PATENTSCHRIFT**

(45) Veröffentlichungstag der Patentschrift:
19.06.91 Patentblatt 91/25

(21) Anmeldenummer: 88890041.2

(22) Anmeldetag: 03.03.88

(51) Int. Cl.⁵: **C02F 3/28**

(54) **Verfahren zum kontinuierlichen anaeroben biologischen Abbau von Verbindungen in Abwässern.**

(30) Priorität: 05.03.87 AT 503/87

(43) Veröffentlichungstag der Anmeldung:
14.09.88 Patentblatt 88/37

(45) Bekanntmachung des Hinweises auf die
Patenterteilung:
19.06.91 Patentblatt 91/25

(84) Benannte Vertragsstaaten:
DE ES FR GR IT

(56) Entgegenhaltungen:
EP-A- 213 691
EP-A- 0 145 792
DE-A- 1 950 787
DE-A- 3 248 703
FR-A- 376 442

(56) Entgegenhaltungen:
WATER RESEARCH, Band 19, Nr. 1, 1985, Seiten 99-106, Pergamon Press Ltd, Exeter, Devon, GB; A. BACHMANN et al.: "Performance
characteristics of the anaerobic baffled reactor"
WATER RESEARCH, Band 19, Nr. 9, 1985, Seiten 1083-1088, Exeter, Devon, GB; S. GHOSH
et al.: "Methane production from industrial
wastes by two-phase anaerobic digestion"

(73) Patentinhaber: FUNDER INDUSTRIE
GESELLSCHAFT M.B.H.
Klagenfurter Strasse 87-89
A-9300 St. Veit a.d. Glan (AT)

(72) Erfinder: Psimenos, Angelos, Dipl.-Ing. Dr.
Altglandorf 16
A-9300 St. Veit/Glan (AT)

(74) Vertreter: Wolfram, Gustav, Dipl.-Ing.
Schwindgasse 7 P.O. Box 205
A-1041 Wien (AT)

## Beschreibung

Die Erfindung betrifft ein Verfahren zum kontinuierlichen anaeroben biologischen Abbau organischer Verbindungen in kommunalen bzw. industriellen Abwässern, wobei die Abwässer mit Schlämmen von selektiv adaptierten Mikroorganismen unter anaeroben Bedingungen in Kontakt gebracht werden und die Abwässer in Mischung mit dem Mikroorganismenschlamm im Gleichstrom durch Abschnitte eines Reaktors geführt werden, wobei die Strömungsrichtung in vertikaler Richtung ein- oder mehrmals umgelenkt wird, so daß in hintereinanderliegenden Abschnitten unterschiedliche Strömungsrichtungen vorhanden sind.

Ein Verfahren dieser Art ist aus Water Research, Band 19, Nr. 1, 1985, Seiten 99-106, bekannt.

Hiebei wird das Abwasser in einem Ein-Stufen-Verfahren abwechselnd durch von oben nach unten durchströmte enge Abschnitte und anschließend in von unten nach oben durchströmte Abschnitte mit gegenüber den ersten Abschnitten stark vergrößertem Volumen geleitet.

Ein Verfahren der eingangs beschriebenen Art ist weiters in der prioritätsälteren nachveröffentlichten europäischen Patentanmeldung 0 213 691 beschrieben.

Bei diesem ebenfalls einstufigen Verfahren stehen sämtliche Abschnitte an den oberen Umlenkungen miteinander in Verbindung und es kommt zu einer Durchmischung des sich oberhalb des Abwassers bildenden Gases.

Bei diesem Verfahren ist keine biologische Filterzone mit vergrößerter Mikroorganismendichte in den unteren Umlenkungsbereichen vorgesehen, sondern es wird eine Durchwirbelung in den einzelnen Kammern, ähnlich wie dies aus Water Research, Band 19, Nr. 1, 1985, Seiten 99-106, bekannt ist, angestrebt.

Aus Water Research, Band 19, Nr. 9, 1985, Seiten 1083-1088, ist ein zweistufiges Verfahren an sich bekannt. Bei diesem zweistufigen Verfahren sind Ein-Kammer-Reaktoren verwendet, in denen eine starke Durchwirbelung der Mikroorganismen mit den zu reinigenden Abwässern stattfindet.

Aus der DE-A 3 345 691 ist es bekannt, in einem eine vertikale Säule bildenden Reaktor ein Festbett aus Trägermaterial zu bilden, an dem Mikroorganismen fixiert sind. Der Reaktor wird von unten nach oben vom Abwasser durchströmt, wobei die 15- bis 20-fache Menge des zulaufenden Abwassers zwischen dem oberen und dem unteren Ende des Reaktors im Kreislauf geführt wird. Bei diesem Verfahren kommt es zu einer Rückvermischung von bereits gereinigtem bzw. teilgereinigtem Abwasser mit frisch zugeführtem Rohabwasser, da eine räumliche Trennung der verschiedenen im Reaktor anfallenden Schritte nicht gegeben ist. Hierdurch ist eine genaue Definition der Verweilzeit des Abwassers im Reaktor, d.h. der Reaktionszeit für die biochemische Reaktion, nicht möglich. Zudem sind im Inneren des Reaktors Einbauten wie ringförmige Einsätze erforderlich, die Kanalbildungen längs der Wände des Festbettes verhindern sollen.

Aus der EP-A-0 071 960 ist eine Anlage zur biologischen Abwasserreinigung bekannt, bei der hintereinander durchströmte Belüftungskammern und ein nachgeordnetes Nachklärbecken vorgesehen sind, wobei mittels eines Schlammverteilers Rücklaufschlamm in die einzelnen Beluftungskammern zugeführt werden kann. Je nach Menge und Beschaffenheit des Rohabwassers werden eine oder mehrere der Belüftungskammern als Kontaktkammern in Kaskadenschaltung in Betrieb genommen. Hierbei ist ein mehr oder weniger langer Aufenthalt des Abwassers in jeder der Belüftungskammern notwendig, so daß eine Rückvermischung von bereits teilweise gereinigtem Abwasser mit dem Rohabwasser erfolgt. Für den Betrieb der Anlage sind weiters Umwälzeinrichtungen innerhalb der Belüftungskammern erforderlich.

Die Erfindung bezweckt die Vermeidung dieser Nachteile und Schwierigkeiten und stellt sich die Aufgabe, ein Verfahren der eingangs beschriebenen Art sowie eine Einrichtung zur Durchführung des Verfahrens zu schaffen, welche jede Rückvermischung gereinigten Abwassers mit Rohabwasser vermeiden, bei denen ein optimaler Kontakt zwischen den Reaktionspartnern, d.h. dem Abwasser und den Mikroorganismen ohne Einbauten, wie z.B. Rührwerke zu erfordern, sichergestellt ist und bei denen die Reaktionszeit für die biochemische Reaktion genau festgelegt werden kann.

Diese Aufgabe wird erfindungsgemäß dadurch gelöst, daß die Abwässer in einer Vorversäuerungsstufe und in einer Methanstufe durch jeweils einen Reaktor mit untereinander gleiche Volumen aufweisenden Abschnitten geführt werden, und in einem unteren bis etwa unter die halbe Höhe der Distanz zwischen unterer und oberer Umlenkung reichenden Umlenkungsbereich eine biologische Filterzone mit vergrößerter Mikroorganismendichte und in den darüberliegenden mittleren und oberen Reaktorbereichen eine Mikroorganismen-Wirbelzone gebildet wird und daß jede obere Umlenkung für sich geschlossen ausgebildet ist und an jeder Umlenkung das entstehende Gas abgeleitet wird.

Dadurch, daß das Abwasser und der rückgeführte Schlamm nur in eine Richtung strömen, ist eine Rückvermischung nicht möglich. Eine Durchmischung des Abwassers mit Hilfe eines Rührwerkes oder mit Hilfe eines eingeblasenen Gases ist nicht erforderlich, da sich im Anschluß an die in den unteren Umlenkungsbereichen vorgesehenen biologischen Filterzonen Wirbelzonen ausbilden, in denen sich eine innige Durchmischung des Abwassers mit den Mikroorganismen zwangsläufig ergibt.

Vorzugsweise wird die Mischung von Abwasser und Mikroorganismenschlamm in der Vorversäuerungs- und Methanstufe auf einem pH-Wert von 4 bis 9 und einer Temperatur von 15 bis 45°C gehalten.

Weiters beträgt zweckmäßig der Trockensubstanzgehalt in der Vorversäuerungsstufe und in der Methanstufe in der biologischen Filterzone 50 bis 100 kg/m³ und in der Mikroorganismen-Wirbelzone 20 bis 50 kg/m³, wobei der organische Anteil der Trockensubstanz zwischen 40 und 80% liegt.

Optimale Ergebnisse lassen sich erzielen, wenn die Raumbelastung in der Vorversäuerungsstufe 10 bis 2000 kg CSB/m³ d und in der Methanstufe 5 bis 100 kg CSB/m³ d beträgt, wobei vorteilhaft die Verweilzeit in der Vorversäuerungsstufe 1 bis 24 Stunden und in der Methanstufe 0,5 bis 8 Tage beträgt.

Hierbei beträgt zweckmäßig der Schlammindexwert in beiden Stufen zwischen 40 und 100 ml/g.

Eine Einrichtung zur Durchführung des Verfahrens mit einem Reaktor mit einer Mehrzahl von jeweils in entgegengesetzten Richtungen durchströmten Abschnitten, die unter Bildung einer mäanderförmigen Durchflußrichtung jeweils im unteren und oberen Bereich miteinander leitungsmäßig verbunden sind, zur Umsetzung des Abwassers mit dem Mikroorganismenschlamm und mit einem Absetzbehälter einer Anlage zum biologischen Abbau von organischen Verbindungen in Abwässern, ist dadurch gekennzeichnet, daß der Reaktor sowohl in der Vorversäuerungs- als auch in der Methanstufe aus einer Mehrzahl von vertikalen Abschnitten mit untereinander gleichen Volumen gebildet ist, wobei das Verhältnis des hydraulischen Durchmessers eines vertikalen Abschnittes des Reaktors zur Summe der Länge aller vertikalen Abschnitte des Reaktors für die Vorversäuerungsstufe mindestens 1 : 10 und für die Methanstufe mindestens 1 : 20 beträgt und daß jede obere Umlenkung für sich geschlossen ausgebildet ist und an jeder oberen Umlenkung eine Gas-Abzugsleitung einmündet.

Eine für kleine Anlagen bevorzugte Ausführungsform ist dadurch gekennzeichnet, daß die vertikalen Abschnitte des Reaktors rohrförmig ausgebildet sind, wobei jeweils zwei benachbarte Rohre U-förmig miteinander verbunden sind.

Eine einfache Bauweise für großvolumige Reaktoren ist dadurch gekennzeichnet, daß der Reaktor als quaderförmiger Behälter ausgebildet ist, der parallel zueinander angeordnete. abwechselnd an der Dekke und am Boden des Behälters befestigte Trennwände aufweist, die abwechselnd einen oberen und einen unteren Umlenkungsbereich bilden.

Zweckmäßig besitzen die einzelnen vertikalen Abschnitte des Reaktors getrennte Zuführungen für Abwasser, Schlamm und Zusätze.

Vorteilhaft ist der untere Teil des dem Reaktor nachgeordneten Absetzbehälters mit einer Rückführleitung für Mikroorganismenschhamm zu den einzelnen vertikalen Abschnitten des Reaktors versehen.

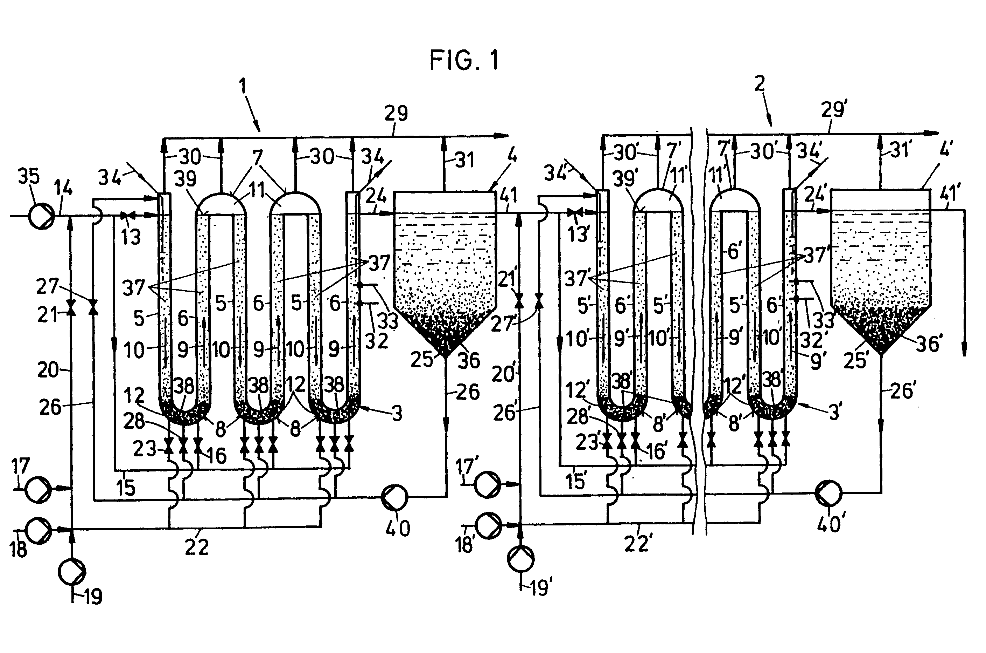

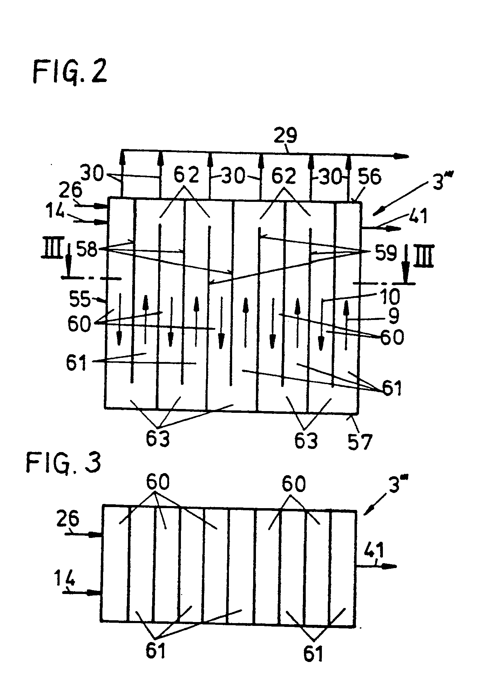

Die Erfindung ist anhand der Zeichnung un mehreren Ausführungsbeispielen näher erläutert, wobei Fig. 1 eine erste Ausführungsform in schematischer Darstellung zeigt. Fig. 2 stellt eine zweite Ausführungsform und Fig. 3 einen Schnitt gemäß der Linie III-III der Fig. 2 dar.

Gemäß Fig. 1 ist eine zweistufige Anlage zum biologischen Abbau organischer Verbindungen in Abwässern mit einer Vorversäuerungsstufe 1 und einer an diese nachfolgend angeordneten Methanstufe 2 gezeigt. Die Vorversäuerungsstufe 1 ist von einem Reaktor 3 und einem nachgeschalteten Absetzbehälter 4 gebildet. Der Reaktor 3 der Vorversäuerungsstufe 1 weist mehrere parallel nebeneinander angeordnete vertikale, rohrförmige Abschnitte 5, 6 mit z.B. kreisförmigem Querschnitt auf, die abwechselnd mit einem oberen und unteren U-förmig ausgebildeten Umlenkungsbogen 7, 8 unter Bildung eines mäanderförmigen Rohrsystems leitungsmäßig miteinander verbunden sind, wodurch ein mäanderförmiger, benachbarte vertikale Bereiche aufweisender Innenraum gebildet ist, der bei Durchfluß abwechselnd nach oben und nach unten gerichtete Strömungsrichtungen, die in den Figuren durch die Pfeile 9, 10 veranschaulicht sind, aufweist, die durch Umlenkungsbereiche 11, 12 in den Umleitungsbögen 7, 8 verbunden sind. Das Verhältnis des hydraulischen Durchmessers eines vertikalen Abschnittes 5 bzw. 6 des Reaktors 3 der Vorversäuerungsstufe 1 zur Summe der Längen aller vertikalen Abschnitte 5, 6 dieses Reaktors beträgt mindestens 1 : 10.

In den oberen Bereich des in Flußrichtung ersten vertikalen Abschnittes 5 des Reaktors 3, in dem die Strömung 10 abwärts gerichtet ist, mündet über ein Absperrorgan 13 eine Zuführleitung 14 für das zu reinigende Abwasser ein, wobei eine von dieser abzweigende Zweigleitung 15 an jeden der unteren Umlenkbögen 8 des Reaktors 3 über Absperrorgane 16 wahlweise anschließbar ist. Die Zweigleitungen 15 könnten auch an jeder Stelle der vertikalen Abschnitte 5, 6 einmünden.

Zuleitungen 17, 18, 19 für Zusätze sind über eine Leitung 20 mit der Abwasserzuführleitung 14 unter Zwischenschaltung eines Absperrorganes 21 leitungsmäßig verbindbar, wodurch eine systemexterne Zudosierung der Zusätze möglich ist. Die Zusätze können auch direkt an jeder Stelle der vertikalen Abschnitte oder in jeden unteren Umlenkungsbogen 8 des Reaktors 3 über eine von der Leitung 20 abzweigende Verteilerleitung 22, die jeweils leitungsmäßig über Absperrorgane 23 mit den vertikalen Abschnitten 5, 6 oder den Umlenkungsbögen 8 verbindbar ist. eingebracht werden.

Der Reaktor 3 der Vorversäuerungsstufe 1 ist mit dem nachgeschalteten Absetzbehälter 4 über eine im oberen Bereich des in Flußrichtung zuletzt ungeordneten vertikalen Abschnittes 6 des Reaktors 3 angeordnete Ableitung 24 leitungsmäßig verbunden. Vom unteren trichterförmigen Ende 25 des Absetzbehälters 4 führt eine Rückführleitung 26 unter Zwischenschaltung eines Absperrorganes 27 zu dem oberen Bereich des in Flußrichtung zuerst angeordneten vertikalen Abschnittes 5 des Reaktors 3, wobei die Rückführleitung 26 wahlweise mit jedem unteren Umlenkungsbogen 8 des Reaktors 3 über Absperrventile 28 leitungsmäßig verbindbar ist. Es wäre auch möglich, die Rückführleitung 26 an beliebigen Stellen der vertikalen Abschnitte 5, 6 einmünden zu lassen. Im oberen Bereich jedes vertikalen Abschnittes 5, 6 bzw. Umlenkbogens 7 ist jeweils eine in eine Gassammelleitung 29 mündende Abzugsleitung 30 angeschlossen. Vom Absetzbehälter 4 führt ebenfalls eine Abzugsleitung 31 in die Gassammelleitung 29.

In dem in Flußrichtung zuletzt angeordneten vertikalen Abschnitt 6 des Reaktors 3 sind Meßeinrichtungen 32, 33 zum Messen der Temperatur und des pH-Wertes des Abwassers angeordnet. Weiters ist im Reaktor 3 eine Beheizungseinrichtung 34 zum Beheizen des Abwassers vorgesehen.

Die der Vorversäuerungsstufe 1 nachgeschaltete Methanstufe 2 ist in gleicher Weise aufgebaut wie die Vorversäuerungsstufe 1 ; dementsprechend weist der Reaktor 3' der Methanstufe 2 die gleichen konstruktiven Merkmale wie der Reaktor 3 der Vorversäuerungsstufe 1 auf, wobei jedoch das Verhältnis des hydraulischen Durchmessers eines vertikalen Abschnittes 5' bzw. 6' des Reaktors 3' der Methanstufe 2 zur Summe der Längen aller vertikalen Abschnitte 5', 6' dieses Reaktors 3' mindestens 1 : 20 beträgt. Zudem ist der Reaktor 3'der Methanstufe 2 wesentlich größer als der Reaktor 3 der Vorversäuerungsstufe 1 dimensioniert.

Das erfindungsgemäße Verfahren ist nachfolgend näher erläutert :

Das zu reinigende Abwasser wird mittels einer Dosierpumpe 35 über die Zuführleitung 14 in den oberen Bereich des ersten vertikalen Abschnittes 5 des Reaktors 3 der Vorversäuerungsstufe 1 eingeleitet, wobei die Abwasserzuführung über die Zweigleitung 15 auch in jeden beliebigen unteren Umlenkungsbereich 8 des Reaktors 3 entsprechend dem biochemischen Reaktionsverlauf und den verfahrenstechnischen Erfordernissen bzw. an beliebig wählbaren Stellen der vertikalen Abschnitte 5, 6 erfolgen kann.

Mikroorganismenschlamm 36 wird entsprechend den Erfordernissen wahlweise über die Rücklaufleitung 26 in den oberen Bereich des ersten vertikalen Abschnittes 5 des Reaktors 3 oder an jeder beliebigen Stelle der vertikalen Abschnitte oder der unteren Umlenkungsbögen 8 des Reaktors 3 eingebracht.

Das Abwasser wird in Mischung mit dem Mikroorganismenschlamm 36 im Gleichstrom durch den Reaktor 3 der Vorversäuerungsstufe 1 geführt, wobei die Strömungsrichtung 9, 10 jeweils in den oberen und unteren Umlenkungsbögen 7, 8 des Reaktors 3 umgelenkt wird. Die hydraulische Durchmischung des Abwasser-Schlammgemisches ergibt sich aus der Durchflußgeschwindigkeit des Abwassers, dem Verhältnis des hydraulischen Durchmessers eines vertikalen Abschnittes 5 bzw. 6 des Reaktors 3 zur Gesamtlänge aller vertikalen Abschnitte 5, 6 des Reaktors 3 sowie der hydraulischen Belastung des Systems.

Im mittleren und oberen Bereich jedes vertikalen Abschnittes 5, 6 entsteht eine Mikroorganismen-Wirbelzone 37. Ein sich in jedem unteren Umlenkungsbogen 8 des Reaktors der Vorversäuerungsstufe 1 absetzender Teil des Mikroorganismenschlammes 36 bildet eine untere biologische Filterzone 38 mit einer gegenüber der Dichte der Mikroorganismen-Wirbelzone 37 im mittleren und oberen Bereich jedes vertikalen Abschnittes 5, 6 vergrößerten Mikroorganismendichte. Diese Wirbelzonen 37 und Filterzonen 38 ermöglichen einen kontinuierlichen anaeroben biologischen Abbau organischer Verbindungen in dem zu reinigenden Abwasser, wobei ein optimaler Kontakt zwischen den Reaktionspartnern, d.h. dem Abwasser und dem Mikroorganismenschlamm 36, sichergestellt ist.

Die organischen Verfbindungen des Abwassers werden dabei in organische Säuren, $H_2$ und $CO_2$ umgesetzt. Die dafür erforderliche Verweilzeit des Abwasser-Schlammgemisches im Reaktor 3 der Vorversäuerungsstufe 1 beträgt je nach Art des Abwassers etwa 1 bis 24 Stunden. Die Konzentrationen der abzubauenden organischen Verbindungen nehmen entlang des Reaktors 3 der Vorversäuerungsstufe 1 ständig ab, wobei eine Rückvermischung von bereits gereinigtem bzw. teilgereinigtem Abwasser mit frisch zugeführtem Rohabwasser bedingt durch die gleichgerichtete Strömung und die baulichen Gegebenheiten des Reaktors 3 nicht möglich ist.

Der biologische anaerobe Abbau der organischen Verbindungen erfolgt bei einem pH-Wert zwischen 4 und 9 und einer Temperatur zwischen 15 und 55°C. Die Einstellung des optimalen Betriebs-pH-Wertes erfolgt durch Zudosieren von Säuren oder Laugen, wie z.B. NaOH, $Ca(OH)_2$, Blaukalk aus der Acetylenherstellung, HCl usw. Diese Zusätze können entsprechend dem Reaktionsverlauf wahlweise im oberen Bereich des ersten vertikalen Abschnittes 5 des Reaktors 3 oder an jeder beliebigen Stelle der vertikalen Abschnitte 5, 6 oder an jeder beliebigen Stelle der vertikalen Abschnitte 5, 6 oder in jedem beliebigen unteren Umlenkungsbereich 12 bzw. Umlenkungsbogen 8 des Reaktors 3 über die Leitung 20 und die Verteilerleitung 22 emgebracht werden.

In analoger Weise erfolgt je nach Bedarf die Zuführung von Nährstoffen und Spurenelementen. Die Messung der Betriebstemperatur und des pH-Betriebswertes erfolgt durch die im Reaktor vorgesehenen Meßeinrichtungen 32, 33, wobei je nach Meßergebnis Korrekturen mit Hilfe der Zusätze bzw. Heizeinrichtung 34 vorgenommen werden.

Das Gemisch aus (teilgereinigtem) Abwasser und Mikroorganismenschlamm 36 gelangt nach Durchströmen des Reaktors 3 der Vorversäuerungsstufe 1 über die Ableitung 24 in den nachgeschalteten Absetzbehälter 4, in dem der Mikroorganismenschlamm 36 durch Sedimentation vom Abwasser getrennt und mittels einer Schlammpumpe 40 über die Rückführleitung 26 wahlweise zum Zulauf oder in jeden beliebigen unteren Umlenkungsbereich 12 des Reaktors 3 bzw. an beliebiger Stelle der vertikalen Abschnitte 5, 6 rückgeführt wird. Das im Absetzbehälter 4 entstehende Biogas wird über die Abzugsleitung 31 abgezogen und in die Gassammelleitung 29 geleitet.

Je nach Durchflußgeschwindigkeit kann der Mikroorganismenschlamm 36 sich entweder in den unteren Umlenkungsbereichen 12 des Reaktors 3 lagern oder mehr oder weniger mit dem durchströmenden Abwasser in den nachgeschalteten Absetzbehälter 4 abgetragen werden, von dem er rückgeführt wird. Über einen Ablauf 41 des Absetzbehälters 4 wird das teilgereinigte Abwasser wahlweise in den oberen Bereich des ersten Vertikalabschnittes 5' oder in die unteren Umlenkungsbereiche 12' bzw. an beliebig wählbarer Stelle der vertikalen Abschnitte 5, 6 des Reaktors 3' der Methanstufe 2 eingeleitet.

Im Reaktor 3' der Methanstufe 2 werden die im teilgereinigten Abwasser enthaltenen organischen Säuren zu Essigsäure und weiter zu Biogas mittels der in den vertikalen Abschnitten 5', 6' und in den unteren Umlenkungsbereichen 12' vorgesehenen biologischen Filterzonen 37', 38' umgesetzt.

Während des Abbaus der organischen Verbindungen in der Methanstufe 2 wird Biogas produziert, das sich in den oberen Bereichen bzw. in den oberen Umlenkungsbögen 7' der vertikalen Abschnitte 5', 6' über dem sich im Reaktor 3' einstellenden Flüssigkeitsspiegel 39' ansammelt. Das Biogas enthält 60 bis 85% Methan und 0,1 bis 1,0% Schwefelwasserstoff, der Rest des Biogases ist Kohlendioxid. Diese Werte stellen jedoch nur einen Mittelwert des gesamten produzierten Biogases dar da die Zusammensetzung des Biogases über die Länge des Reaktors 3' unterschiedlich ist. Das Biogas wird über die Abzugsleitungen 30' in die Gassammelleitung 29' abgezogen und der weiteren Verwertung zugeführt. Die dafür erforderliche Verweilzeit beträgt je nach Art des Abwassers 0,5 bis 8 Tage. Eine pH-Wert-Einstellung sowie eine Zudosierung von Nährlösungen und Spurenelementen ist im Reaktor 3' der Methanstufe 2 nicht erforderlich ; es ist jedoch zweckmäßig, die dafür erforderlichen Einrichtungen beim Bau der Anlage vorzusehen.

Im Absetzbehälter 4' der Methanstufe 2 wird der Mikroorganismenschlamm 36' durch Sedimentation vom Abwasser getrennt und in analoger Weise zur Vorversäuerungsstufe 1 in den Reaktor 3' der Methanstufe 2 rückgeführt, wobei das im Absetzbehälter 4' entstehende Biogas abgezogen und in die Gassammelleitung 29' eingeleitet wird. Das gereinigte Abwasser wird über den Ablauf 41' des Absetzbehälters 4' einem Vorfluter oder einer Restreinigung zugeführt.

Die Raumbelastung beträgt je nach Art bzw. Zusammensetzung des Abwassers in der Vorversäuerungsstufe 1 10 bis 2000 kg CSB/$m^3$d (CSB = Chemischer Sauerstoffbedarf) und in der Methanstufe 2 5 bis 100 kg CSB/$m^3$d. Der Trockensubstanzgehalt beträgt in beiden Stufen in den Mikroorganismen-Wirbelzonen 37, 37' 20 bis 50 kg/$m^3$ und in den unteren Filterzonen 38, 38' 50 bis 100 kg/$m^3$.

Der organische Anteil der Trockensubstanz liegt jeweils bei 40 bis 80%. Die Schlammindexwerte beider Stufen 1, 2 liegen zwischen 40 und 100 ml/g.

Die in den Fig. 2 und 3 gezeigte Ausführungsform ist besonders für großvolumige Reaktoren sowohl für die Vorversäuerungsstufe 1 als auch die Methanstufe 2 geeignet. Der Reaktor 3''' ist von einem quaderförmigen Behälter 55 gebildet, an dessen Decken- und Bodenbereich 56, 57 abwechselnd parallel zueinander angeordnete und zur gegenüberliegenden Wand einen Spalt freilassende Trennwände 58, 59 befestigt sind, so daß mehrere hintereinanderliegende Vertikalabschnitte 60, 61 abwechselnd einen oberen und einen unteren Umlenkungsbereich 62, 63 bilden. Das Abwasser-Schlammgemisch durchströmt den Reaktor 3''' in zu der in Fig. 1 gezeigten Ausführungsform analoger Weise.

Die Erfindung beschränkt sich nicht auf die in der Zeichnung dargestellten Ausführungsformen, sondern kann in verschiedener Hinsicht modifiziert werden. So können z.B. Ableitungen 24 für das Abwasser-Schlammgemisch nicht nur am Ende des Reaktors, sondern auch in den einzelnen vertikalen Abschnitten 5, 6 vorgesehen sein, die jeweils entsprechend der Menge und Zusammensetzung des zu reinigenden Abwassers geöffnet bzw. geschlossen sind, wodurch nur die für eine ausreichende Reinigung des Abwassers notwendige Anzahl von vertikalen Abschnitten 5, 6 in Betrieb ist und die in Flußrichtung dahinter liegenden vertikalen Abschnitte 5, 6 nicht durchströmt werden. Weiters kann die Querschnittsfläche und -form jedes einzelnen vertikalen Abschnittes 5, 6 je nach Bedarf gegenüber den anderen vertikalen Abschnitten 5, 6 variiert werden.

Das erfindungsgemäße Verfahren und die gezeigten Einrichtungen zur Durchführung des Verfahrens ermöglichen bei einfacher Bauweise des Reaktors 3 eine Verkleinerung des Reaktorvolumens der Vorversäuerungsstufe 1 auf mindestens ein Drittel des bei bekannten Einrichtungen benötigten Volumens, wodurch eine erhebliche Verminderung der Baukosten und eine beträchtliche Platzersparnis möglich ist. Für den Betrieb der Anlage ist eine nur geringe Anzahl von Pumpen notwendig, und es müssen keine Rühreinrichtungen oder sonstige Einbauten im Reaktor 3 vorgesehen werden, wodurch neben den Investitionskosten auch die Betriebskosten gering gehalten werden können. Vorteilhaft ist neben der hohen Leistungsfähigkeit des Reaktors 3 die Unempfindlichkeit der Anlage gegen Störungen bzw. Substrat- und pH-Stöße. Weiters ist bei der erfindungsgemäßen Einrichtung die Einstellung des pH-Wertes des Abwassers in einfacher Weise durchführbar, und es kann eine genau definierte Reaktionszeit für die biochemische Reaktion bzw. für die erforderliche Verweilzeit des Abwassers im Reaktor 3 eingehalten werden.

Bei dem erfindungsgemäßen Verfahren kommt es zu einer schonenden Vermischung des Abwassers mit dem Mikroorganismenschlamm 36, die für die anaerobe Biozönose vorteilhaft ist, da die Mikroorganismen keine intensive Vermischung vertragen.

Weiters schaden biologisch hemmende Substratstöße des Abwassers wenigf da diese sich als "Segment" durch den Reaktor 3 bewegen und es nicht zu einer Vermischung mit der gesamten Biozönose kommt.

Beispiel 1 :

Ein Abwassergemisch, bestehend aus Molke und Kommunalabwasser, wird mittels der Dosierpumpe 35 in den Reaktor 3 der Vorversäuerungsstufe 1 einer entsprechend Fig. 1 gestalteten Anlage eingeleitet. Der CSB-Gehalt des Abwassers beträgt 20.000 mg/l und der Gehalt an organischen Säuren 40 mMol/l.

Das Reaktorvolumen in der Vorversäuerungsstufe 1 beträgt 20 l und die zudosierte Menge an zu reinigendem Abwasser 5 bis 20 l/h. Daraus ergibt sich eine mittlere Verweilzeit im Reaktor 3 der Vorversäuerungsstufe 1 von 1 bis 4 Stunden. Die Dosierung des Abwassergemisches erfolgt am Anfang des Reaktors 3. Das Rücklaufschlammverhältnis in der Vorversäuerungsstufe 1 beträgt 50 bis 100%.

Entlang des Reaktors 3 der Vorversäuerungsstufe 1 wird der CSB-Gehalt und der Gehalt der organischen Säuren mittels nicht dargestellter Meßeinrichtungen gemessen.

Die pH-Wert-Einstellung des zu reinigenden Abwassers erfolgt extern am Anfang des Reaktors 3 der Vorversäuerungsstufe 1 mittels NaOH auf einen pH-Wert von 11. Diese pH-Wert-Einstellung ermöglicht es, am Ende des Reaktors 3 der Vorversäuerungsstufe 1 einen pH-Wert von 6,5 bis 7,3 zu erreichen. Phosphor- und Stickstofflösungen sowie Spurenelemente werden nicht zugesetzt, wenn die im Abwasser vorhandene Konzentration ausreichend ist.

Der CSB-Gehalt bei der Verweilzeit von 4 Stunden bleibt bis zu 2/3 der Reaktorlänge konstant und nimmt gegen Ende des Reaktors 3 ab. Ähnliches kann man für den Gehalt der organischen Säuren beobachten. In der Mitte des Reaktors 3 beträgt die Konzentration der organischen Säuren 200 mMol/l, bei einer Länge von 2/3 des Reaktors beträgt die Konzentration der organischen Säuren 270 mMol/l und am Ende des Reaktors 3 180 mMol/l. Die Raumbelastung der Vorversäuerungsstufe 1 beträgt bei einer Verweilzeit von 4 Stunden 120 kg CSB/m³d und bei 1 Stunde 480 kg CSB/m³d. Bei einer parallel betriebenen Rührkessel-Vorversäuerung wird bei einer Verweilzeit von über 5 Stunden nur eine geringe Säurekonzentration von 50 mMol/l erreicht.

Im Anschluß an die Vorversäuerungsstufe 1 wird das teilgereinigte Abwasser in die Methanstufe 2 eingeleitet.
Das Reaktorvolumen dieser Stufe beträgt 480 l.
Die Verweilzeit beträgt bei 5 l/h 4 Tage und bei 120 l/h 1 Tag.
Die Raumbelastung beträgt
bei einer Verweilzeit von 4 Tagen : 4,50 kg CSB/m³d
bei einer Verweilzeit von 1 Tag : ca. 18,0 kg CSB/m³d.

Die Abbaurate beträgt etwa 93 bis 98%. Das produzierte Biogas enthält 75 bis 80% $CH_4$ 0,1 bis 0,4% $H_2S$, der Rest ist $CO_2$.

Die Temperatur des Systems Abwasser-Schlammgemisch beträgt 28°C im Reaktor 3 der Vorversäuerungsstufe 1 und 30°C im Reakor 3' der Methanstufe 2.

Beispiel 2 :

Ein Abwasser aus der Hartfaserplattenproduktion mit einem CSB-Gehalt von 15.000 bis 20.000 mg/l wird in der gemäß Beispiel 1 verwendeten Anlage gereinigt.

Der pH-Wert des zu reinigenden Abwassers wird auf 11 eingestellt. Die pH-Einstellung erfolgt mit Blaukalk aus der Acetylenproduktion. Damit erreicht man am Ende der Vorversäuerungsstufe 1 einen pH-Wert des Abwasser-Schlammgemisches von 6,6 bis 7,4. In der Methanstufe 2 ist eine neue pH-Wert-Einstellung nicht mehr notwendig. Als N-Quelle wird Harnstoff und als P-Quelle $H_3PO_4$ verwendet. Dazu werden Spurenelemente zugesetzt.

Die Verweilzeit variiert zwischen 1 und 10 Stunden in der Vorversäuerungsstufe 1 und zwischen 1 und 10 Tagen in der Methanstufe 2. Die Raumbelastung des Reaktors 3 beträgt in der Vorversäuerungsstufe 136 bis 480 kg CSB/m³d und in der Methanstufe 22 bis 18 kg CSB/m³d. Die Abbaurate beträgt etwa 80 bis 93%. Der Methangehalt des produzierten Biogases beträgt 70 bis 80%, der $H_2S$-Gehalt etwa 0,1 bis 0,6%.

Beispiel 3 :

In der gemäß Beispiel 1 bzw. 2 verwendeten Anlage wird eine künstliche Lösung aus Rübenzukker, Harnstoff, $H_3PO_4$ und Spurenelementen sowie 1% Molke mit einem CSB-Wert von 30.000 bis 40.000 mg/l gereinigt.

Die pH-Wert-Einstellung erfolgt vor der Einleitung des Abwassers in den Reaktor 3 der Vorversäuerungsstufe 1 auf einen pH-Wert von etwa 11,5 bis 12 mittels einer Brandkalksuspension.

Es werden Verweilzeiten im Reaktor 3 der Vorversäuerungsstufe 1 zwischen 0,5 und 6 Stunden und im Reaktor 3' der Methanstufe 2 zwischen 0,5 und 6 Tagen erzielt.

Die Raumbelastung beträgt im Reaktor 3 der Vorversäuerungsstufe 1 160 bis 1.900 kg $CSB/m^3d$ und im Reaktor 3' der Methanstufe 2 6 bis 70 kg $CSB/m^3d$.

Die Abbaurate der Anlage beträgt 85 bis 90%. Das produzierte Biogas besteht aus 80 bis 87% $CH_4$ 0,0 bis 0,1 $H_2S$ und der Rest aus $CO_2$.

## Ansprüche

1. Verfahren zum kontinuierlichen anaeroben biologischen Abbau organischer Verbindungen in kommunalen bzw. industriellen Abwässern, wobei die Abwässer mit Schlämmen (36, 36') von selektiv adaptierten Mikroorganismen unter anaeroben Bedingungen in Kontakt gebracht werden und die Abwässer in Mischung mit dem Mikroorganismenschlamm (36, 36') im Gleichstrom durch Abschnitte (5, 6, 5', 6') eines Reaktors (3, 3', 3''') geführt werden, wobei die Strömungsrichtung (9, 9', 10, 10') in vertikaler Richtung ein- oder mehrmals umgelenkt wird, so daß in hintereinanderliegenden Abschnitten unterschiedliche Strömungsrichtungen vorhanden sind, dadurch gekennzeichnet, daß die Abwässer in einer Vorversäuerungsstufe (1) und in einer Methanstufe (2) durch jeweils einen Reaktor mit untereinander gleiche Volumen aufweisenden Abschnitten geführt werden, und in einem unteren, Umlenkungsbereich (12, 12', 63) eine biologische Filterzone (38, 38') mit vergrößerter Mikroorganismendichte und in den darüberliegenden mittleren und oberen Reaktorbereichen eine Mikroorganismen-Wirbelzone (37, 37') gebildet wird und daß jede obere Umlenkung für sich geschlossen ausgebildet ist und an jeder Umlenkung das entstehende Gas abgeleitet wird.

2. Verfahren nach Anspruch 1, dadurch gekennzeichnet, daß die Mischung von Abwasser und Mikroorganismenschlamm (36, 36') in der Vorversäuerungs- (1) und Methanstufe (2) auf einem pH-Wert von 4 bis 9 und einer Temperatur von 15 bis 45°C gehalten wird.

3. Verfahren nach Anspruch 1 oder 2, dadurch gekennzeichnet, daß der Trockensubstanzgehalt in der Vorversäuerungsstufe (1) und in der Methanstufe (2) in der biologischen Filterzone (38, 38') 50 bis 100 kg/$m^3$ und in der Mikroorganismen-Wirbelzone (37, 37') 20 bis 50 kg/$m^3$ beträgt, wobei der organische Anteil der Trockensubstanz zwischen 40 und 80% liegt.

4. Verfahren nach einem oder mehreren der Ansprüche 1 bis 3, dadurch gekennzeichnet, daß die Raumbelastung in der Vorversäuerungsstufe (1) 10 bis 2000 kg $CSB/m^3$ d und in der Methanstufe (2) 5 bis 100 kg $CSB/m^3$ d beträgt.

5. Verfahren nach einem oder mehreren der Ansprüche 1 bis 4, dadurch gekennzeichnet, daß die Verweilzeit in der Vorversäuerungsstufe (1) 1 bis 24 Stunden und in der Methanstufe (2) 0,5 bis 8 Tage beträgt.

6. Verfahren nach einem oder mehreren der Ansprüche 1 bis 5, dadurch gekennzeichnet, daß der Schlammindexwert in beiden Stufen zwischen 40 und 100 ml/g beträgt.

7. Einrichtung zur Durchführung des Verfahrens nach einem oder mehreren der Ansprüche 1 bis 6, mit einem Reaktor (3, 3', 3''') mit einer Mehrzahl von jeweils in entgegengesetzten Richtungen durchströmten Abschnitten (5, 6, 5', 6', 60, 61), die unter Bildung einer mäanderförmigen Durchflußrichtung jeweils im unteren und oberen Bereich miteinander leitungsmäßig verbunden sind, zur Umsetzung des Abwassers mit dem Mikroorganismenschlamm (36, 36') und mit einem Absetzbehälter (4, 4') einer Anlage zum biologischen Abbau von organischen Verbindungen in Abwässern, dadurch gekennzeichnet, daß der Reaktor (3, 3', 3''') sowohl in der Vorversäuerungs- (1) als auch in der Methanstufe (2) aus einer Mehrzahl von vertikalen Abschnitten mit untereinander gleichen Volumen gebildet ist, wobei das Verhältnis des hydraulischen Durchmessers eines vertikalen Abschnittes (5, 6, 5', 6', 60, 61) des Reaktors (3, 3', 3''') zur Summe der Länge aller vertikalen Abschnitte (5, 6, 5', 6', 60, 61) des Reaktors (3, 3', 3''') für die Vorversäuerungsstufe (1) mindestens 1 : 10 und für die Methanstufe (2) mindestens 1 : 20 beträgt und daß jede obere Umlenkung für sich geschlossen ausgebildet ist und an jeder oberen Umlenkung eine Gas-Abzugsleitung (30, 30') einmündet.

8. Einrichtung nach Anspruch 7, dadurch gekennzeichnet, daß die vertikalen Abschnitte (5, 6, 5', 6') des Reaktors (3, 3') rohrförmig ausgebildet sind, wobei jeweils zwei benachbarte Rohre (5, 6, 5', 6') U-förmig miteinander verbunden sind.

9. Einrichtung nach Anspruch 7, dadurch gekennzeichnet, daß der Reaktor (3'') als quaderförmiger Behälter (55) ausgebildet ist, der parallel zueinander angeordnete, abwechselnd an der Decke (56) und am Boden (57) des Behälters (55) befestigte Trennwände (58, 59) aufweist, die abwechselnd einen oberen und einen unteren Umlenkungsbereich (62, 63) bilden.

10. Einrichtung nach einem oder mehreren der Ansprüche 7 bis 9, dadurch gekennzeichnet, daß die einzelnen vertikalen Abschnitte (5, 6, 5', 6') des Reaktors (3, 3') getrennte Zuführungen (14, 15, 20, 22, 26, 14', 15', 20', 22', 26') für Abwasser, Schlamm und Zusätze besitzen.

11. Einrichtung nach einem oder mehreren der Ansprüche 7 bis 10, dadurch gekennzeichnet, daß der untere Teil (25, 25') des dem Reaktor (3, 3') nachgeordneten Absetzbehälters (4, 4') mit einer Rückführleitung (26, 26') für Mikroorganismenschlamm (36, 36') zu den einzelnen vertikalen Abschnitten (5, 6, 5', 6') des Reaktors (3, 3') versehen ist.

## Claims

1. A process for the continuous anearobic biological degradation of organic compounds in communal and industrial waste waters by contacting the waste waters with slurries (36, 36') of selectively adapted microorganisms under anaerobic conditions and conducting the waste waters mixed with the microorganism slurry (36, 36') in the co-current flow through sections (5, 6, 5', 6') of a reactor (3, 3', 3''') by deflecting the flow direction (9, 9', 10, 10') once or several times in the vertical sense such that different flow directions prevail in consecutive sections, characterized in that the waste waters are conducted in a pre-acidification stage (1) and in a methane stage (2) through one reactor each, which reactor comprises sections of equal volume, and a biological filtering zone (38, 38') having an elevated microorganism density is formed in a lower deflection region (12, 12', 63) and a microorganism whirling zone (37, 37') is formed in the superimposed, central and upper regions of the reactor, and that each upper deflection is designed as a unit and the gas forming at each deflection is conducted away.

2. A process according to claim 1, characterized in that the mixture of waste water and microorganism slurry (36, 36') is maintained at a pH of from 4 to 9 and at a temperature of from 15 to 45°C in the pre-acidification (1) and methane (2) stages.

3. A process according to claim 1 or 2, characterized in that the dry substance content in the pre-acidification stage (1) and in the methane stage (2) amounts to 50 to 100 kg/m³ in the biological filtering zone (38, 38') and to 20 to 50 kg/m³ in the microorganism whirling zone (37, 37'), the organic portion of the dry substance ranging between 40 and 80%.

4. A process according to one or several of claims 1 to 3, characterized in that the space load is 10 to 2,000 kg COD/m³d in the pre-acidification stage (1) and 5 to 100 kg COD/m³d in the methane stage (2).

5. A process according to one or several of claims 1 to 4, characterized in that the residence time is 1 to 24 hours within the pre-acidification stage (1) and 0.5 to 8 days within the methane stage (2).

6. A process according to one or several of claims 1 to 5, characterized in that the slurry index value amounts to between 40 and 100 ml/g in both stages.

7. An arrangement for carrying out the process according to one or several of claims 1 to 6, comprising a reactor (3, 3', 3''') including a plurality of sections (5, 6, 5', 6', 60, 61) each passed in opposite directions and flow-connected with each other in the lower and upper regions by forming a meander-like flow direction for the reaction of the waste waters with the microorganism slurry (36, 36') and a settling tank (4, 4') of an installation for the biological degradation of organic compounds in waste waters, characterized in that the reactor (3, 3', 3'''), both in the pre-acidification stage (1) and in the methane stage (2), is comprised of a plurality of vertical sections having equal volumes, wherein the ratio of the hydraulic diameter of a vertical section (5, 6, 5', 6', 60, 61) of the reactor (3, 3', 3''') to the sum of the lengths of all the vertical sections (5, 6, 5', 6', 60, 61) of the reactor (3, 3', 3''') is at least 1 : 10 for the pre-acidification stage (1) and is at least 1 : 20 for the methane stage (2), and that each upper deflection is designed as a unit, a gas-discharge duct (30, 30') running into each upper deflection.

8. An arrangement according to claim 7, characterized in that the vertical sections (5, 6, 5', 6') of the reactor (3, 3') are designed as tubes, two neighbouring tubes (5, 6, 5', 6') each being connected in a U-shaped manner.

9. An arrangement according to claim 7, characterized in that the reactor (3''') is designed as a parallelepipedic vessel (55) including parallely arranged partition walls (58, 59) fastened alternately to the ceiling (56) and to the bottom (57) of the vessel (55) and alternately forming an upper and a lower deflection region (62, 63).

10. An arrangement according to one or several of claims 7 to 9, characterized in that the individual vertical sections (5, 6, 5', 6') of the reactor (3, 3') are equipped with separate feeds (14, 15, 20, 22, 26, 14', 15', 20', 22', 26') for waste water, slurry and additives.

11. An arrangement according to one or several of claims 7 to 10, characterized in that the lower part (25, 25') of the settling reservoir (4, 4') following the reactor (3, 3') is provided with a return duct (26, 26') for microorganism slurry (36, 36') leading to the individual vertical sections (5, 6, 5', 6') of the reactor (3, 3').

## Revendications

1. Procédé de biodégradation anaérobie continue de composés organiques dans des eaux usées communales ou industrielles, les eaux usées étant mises en contact avec des boues (36, 36') de microorganismes adaptés sélectivement dans des conditions anaérobies et les eaux usées mélangées aux boues de micro-organismes (36, 36′) étant entraînées dans un même courant dans les tronçons (5, 6, 5′, 6′) d'un réacteur (3, 3′, 3‴), le sens d'écoulement (9, 9′, 10, 10′) en direction verticale étant modifié une ou plusieurs fois, de sorte que des tronçons successifs présentent des sens d'écoulement différents, caractérisé en ce que les eaux usées sont entraînées au cours d'une étape de pré-acidification (1) et d'une étape de méthane (2) dans un réacteur comportant des tronçons de volumes identiques et qu'est formée dans une zone inférieure de changement de direction (12, 12′, 63) une zone filtre biologique (38, 38′) présentant une densité en micro-organismes supérieure et dans les zones moyennes et supérieures sus-jacentes du réacteur une zone de tourbillonnement des micro-organismes (37, 37′), que chaque changement de direction supérieur forme une unité en soi et que le gaz formé est évacué à chaque changement de direction.

2. Procédé selon la revendication 1, caractérisé en ce que le mélange des eaux usées et des boues de micro-organismes (36, 36′) est maintenu au cours de l'étape de pré-acidification (1) et de l'étape de méthane (2) à un pH compris entre 4 et 9 et à une température comprise entre 15 et 45°C.

3. Procédé selon la revendication 1 ou 2, caractérisé en ce que la teneur en matière sèche au cours de l'étape de pré-acidification (1) et de l'étape de méthane (2) est comprise entre 50 et 100 kg/m³ dans la zone filtre biologique (38, 38′) et entre 20 et 50 kg/m³ dans la zone de tourbillonnement des micro-organismes (37, 37′), le pourcentage de substances organiques dans la matière sèche étant compris entre 40 et 80%.

4. Procédé selon une ou plusieurs des revendications 1 à 3, caractérisé en ce que la charge est comprise entre 10 et 2000 kg de DCO/m³ d au cours de l'étape de pré-acidification (1) et entre 5 et 100 kg de DCO/m³ d au cours de l'étape de méthane (2).

5. Procédé selon une ou plusieurs des revendications 1 à 4, caractérisé en ce que le temps de séjour est compris entre 1 et 24 h au cours de l'étape de pré-acidification (1) et entre 0,5 à 8 j au cours de l'étape de méthane (2).

6. Procédé selon une ou plusieurs des revendications 1 à 5, caractérisé en ce que l'index de boues est compris entre 40 et 100 ml/g au cours des deux étapes.

7. Dispositif de mise en oeuvre du procédé selon une ou plusieurs des revendications 1 à 6, comprenant un réacteur (3, 3′, 3‴) comportant une pluralité de tronçons (5, 6, 5′, 6′, 60, 61) parcourus en sens contraires, reliés entre eux à leurs parties supérieures et inférieures par des conduites formant un sens d'écoulement en zigzag, destiné à faire réagir les eaux usées avec les boues de micro-organismes (36, 36′) ainsi qu'un décanteur (4, 4′) d'une station de biodégradation de composés organiques dans des eaux usées, caractérisé en ce que le réacteur (3, 3′, 3‴) est constitué tant au cours de l'étape de pré-acidification (1) qu'au cours de l'étape de méthane (2) par une pluralité de tronçons verticaux de mêmes volumes, le rapport entre le diamètre hydraulique d'un tronçon vertical (5, 6, 5′, 6′, 60, 61) du réacteur (3, 3′, 3‴) et la longueur totale de tous les tronçons verticaux (5, 6, 5′, 6′, 60, 61) du réacteur (3, 3′, 3‴) étant au minimum de 1 : 10 pour l'étape de pré-acidification (1) et au minimum de 1 : 20 pour l'étape de méthane (2), que chaque changement de direction supérieur constitue une unité en soi et qu'une conduite d'évacuation de gaz (30, 30′) débouche dans chaque changement de direction supérieur.

8. Dispositif selon la revendication 7, caractérisé en ce que les tronçons verticaux (5, 6, 5′, 6′) du réacteur (3, 3′) sont tubulaires, deux tubes voisins (5, 6, 5′, 6′) étant réunis en formant un U.

9. Dispositif selon la revendication 7, caractérisé en ce que le réacteur (3‴) a la forme d'un réservoir parallélépipédique (55) présentant des cloisons (58, 59) parallèles, fixées alternativement au plafond (56) et au sol (57) du réservoir (55), formant alternativement une zone supérieure et une zone inférieure de changement de direction (62, 63).

10. Dispositif selon une ou plusieurs des revendications 7 à 9, caractérisé en ce que les différents tronçons verticaux (5, 6, 5′, 6′) du réacteur (3, 3′) possèdent des conduites d'amenée distinctes (14, 15, 20, 22, 26, 14′, 15′, 20′, 22′, 26′) pour les eaux usées, les boues et les additifs.

11. Dispositif selon une ou plusieurs des revendications 7 à 10, caractérisé en ce que la partie inférieure (25, 25′) du décanteur (4, 4′) situé en aval du réacteur (3, 3′) est munie d'une conduite de recyclage (26, 26′) des boues de micro-organismes (36, 36′) aux différents tronçons verticaux (5, 6, 5′, 6′) du réacteur (3, 3′).

FIG. 1

FIG. 2

FIG. 3